# FASCICULE DE BREVET EUROPEEN

(11) **EP 0 593 831 B1**
(45) Date de publication et mention de la délivrance du brevet: **04.05.1994**
(21) Numéro de dépôt: 92440127.6
(22) Date de dépôt: 18.11.1992
(51) Int. Cl.: C07C 403/08, C07C 31/135, C07C 33/025, A61K 31/045

(54) **Alcools gras à longue chaîne à action neurothrophique et promnesiante**
Langkettige Fettalkohole mit neurotropischer und gedächtnissfördernder Wirkung
Long-chain fatty alcohols as neurotrophic and memory improving agents

(43) Date de publication de la demande: 27.04.1994
(73) Titulaire: MEDAFOR, F-67400 Illkirch Graffenstaden (FR)
(72) Inventeur: Luu, Bang, F-67000 Strasbourg (FR); Borg, Jacques, F-67800 Bischheim (FR); Beck, Alain, F-67400 Illkirch (FR); Laabich, Aicha, F-67400 Illkirch (FR); Crestani, Florence, F-67400 Illkirch (FR); Neveux, Odile, F-67800 Bischheim (FR)
(74) Mandataire: Bossard, Jacques-René

(56) Documents cités:
- WO-A-91/05754
- FR-A- 78 168
- US-A- 3 578 686

## Description

La présente invention concerne un nouveau groupe d'alcools gras à longue chaîne possédant des propriétés utiles dans le traitement des maladies neurodégénératives et pour la récupération fonctionnelle à la suite de lésions du tissu nerveux ou au cours du vieillissement cérébral ou encore pour améliorer les fonctions cognitives chez un sujet sain, en particulier sa mémoire.

La Déposante a déjà décrit dans le brevet PCT N° WO 91/05754 une large famille de dérivés d'alcools gras dans laquelle un sous-groupe est constitué d'une chaine hydrocarbonée linéaire comprenant 7 à 25 atomes de carbone, comportant éventuellement des doubles liaisons et portant à une extrémité une fonction alcool primaire et à l'autre extrémité un radical cyclohexyle substitué par des groupes méthyles, ce sous-groupe répondant à la formule générale: 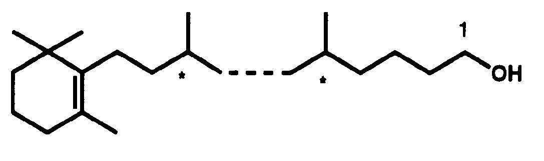
ou

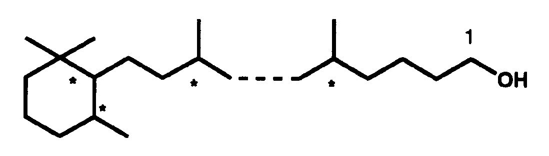

Les membres de ce sous-groupe présentent des propriétés biologiques intéressantes dans la mesure où l'expérimentation *in vitro* a démontré qu'ils favorisent la différenciation et la survie neuronale et *in vivo* ils sont capables de compenser les déficits comportementaux aprés lésion cérébrale.

Ils peuvent être présentés sous forme de préparations pharmaceutiques utilisables en médecine humaine pour le traitement de nombreuses affections, qui sont énumérées en détail dans le WO 91/05754 précité.

Le nouveau groupe de composés selon l'invention est constitué par les composés de formule générale: 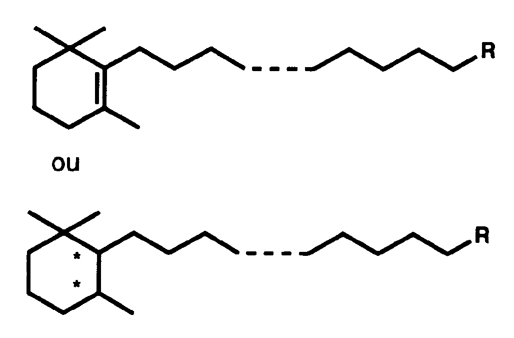
avec
**R**= -CH₂CH₂CH₂OH
ou
**R**= -CH=CHCH₂OH, *cis* ou *trans*
dans laquelle ladite chaîne comporte 7 à 25 atomes de carbone, et est saturée ou comporte une double liaison de configuration *cis* ou *trans* en β de la fonction alcool primaire.

Cette particularité représente un avantage considérable, dans la mesure où l'absence complète ou même seulement la diminution du nombre de carbones asymétriques réduit considérablement les difficultés de synthèse conduisant à l'obtention de produits optiquement purs.

A titre d'exemple les composés A₁, A₂ et A₃, appartenant à ce groupe, et de formule: 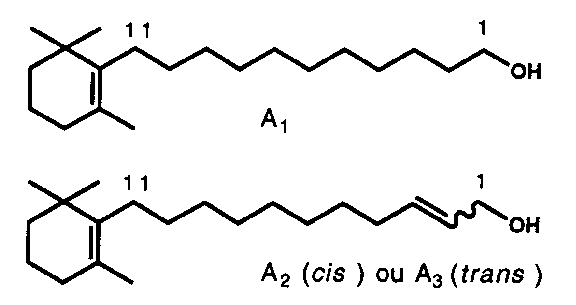
ne comportent qu'un seul isomère théorique.

De même, les composés B₁, B₂ et B₃, appartenant à ce groupe, et de formule: 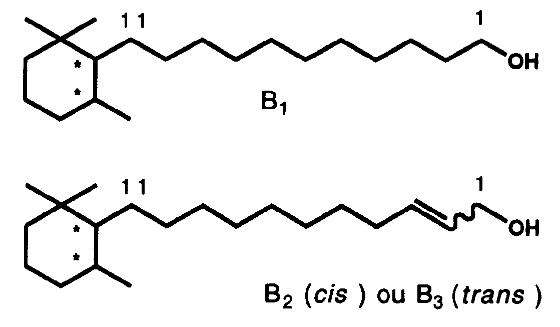
ne comportent que quatre isomères théoriques et deux en pratique, dans le cas d'une hydrogénation catalytique à partir des précurseurs de type A correspondant.

Ce nombre d'isomères est très faible si on le compare par exemple à celui des isomères du composé le plus voisin de B₁, mais portant deux groupes méthyles en position 5 et 9, qui est de 16. Cette limitation des problèmes de stéréochimie a pour avantage, outre la réduction des difficultés de synthèse et l'accroissement de la pureté des produits, de limiter le nombre d'études biologiques, et corollairement d'augmenter les chances d'enregistrement de ces produits par les autorités administratives dans les pays où l'on envisage l'exploitation commerciale de ces produits.

Les composés selon l'invention favorisent la différenciation (extension de neurites) et la survie neuronale *in vitro* chez le rat. Ils peuvent donc être utiles dans le traitement des maladies neurodégénératives et pour la récupération fonctionnelle à la suite de lésions traumatiques ou chimiques du tissu nerveux. Par ailleurs il a été constaté que, outre leur effet neurotrophique sur des sujets malades ou vieillissants, les nouveaux composés selon l'invention exercent un effet neurotrope sur des sujets sains et normaux, favorisant et améliorant leurs capacités cognitives.

L'invention concerne donc également les compositions pharmaceutiques comprenant au moins un des dérivés selon l'invention, aux doses efficaces de l'ordre de 0,1 à 100 mg/kg (administration unique ou répétée) , notamment de 5 à 50 mg/kg . Les compositions pharmaceutiques de l'invention sont administrées par voie parentérale ou par voie orale. Cette dernière est la voie préférable et permet d'obtenir un bon passage cérébral à un taux compatible avec l'effet thérapeutique recherché.

Le rapport cerveau/sang est nettement supérieur à un, 6 heures après l'administration, et témoigne d'une bonne affinité des molécules pour le tissu nerveux. La concentration maximale est obtenue dans le cerveau entre 6 et 24 heures après l'administration orale.

D'autres caractéristiques de l'invention apparaîtront au cours de la description qui suit des processus de synthèse des composés A₁ et B₁, pris à titre d'exemple, ainsi que de l'exposé des essais biologiques *in vitro* auxquels il a été procédé.

### I) Synthèses chimiques des composés de l'invention.

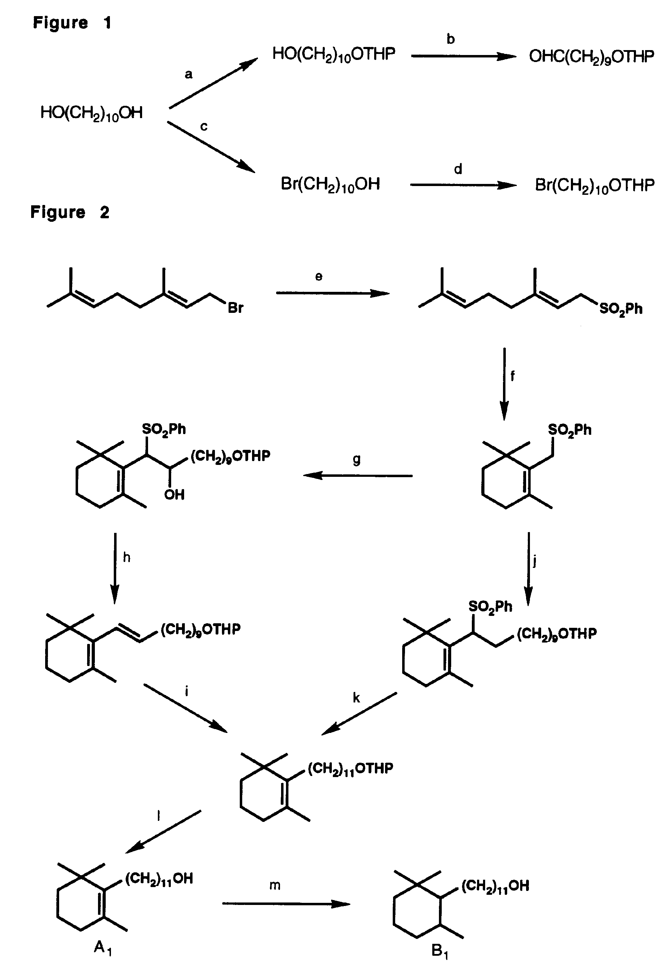

Le processus de synthèse des composés A₁ et B₁ est illustré par les **figures 1** et **2** du dessin annexé, qui schématisent les deux séries d'étapes constituant l'ensemble de ce processus, à savoir:
- les étapes a et b consistent à préparer en premier lieu un synthon linéaire, à partir du 1,10-décanediol, qui à l'étape a est protégé par la formation de l'éther de tétrahydropyrannyle et dont la fonction alcool libre restante est oxydée en aldéhyde à l'étape b.
- les étapes c et d consistent à préparer un autre synthon linéaire, permettant de préparer les composés de type A et B de manière plus directe. Le 1,10-décanediol est bromé à I'étape c en présence d'acide bromhydrique et la fonction alcool libre est protégée par la formation de l'éther de tétrahydropyrannyle à l'étape d.
- les étapes e à m consistent à préparer l'autre partie des molécules, à partir du bromure de géranyle, substitué en sulfone à l'étape e, que l'on cyclise à l'étape f.

La sulfone cyclisée est alors:
1) soit (étape g) couplée à l'aldéhyde obtenu à l'étape b, pour obtenir l'hydroxysulfone. Par élimination à l'étape h on obtient le composé diénique correspondant, dont la double liaison (exocyclique) est réduite par hydrogénation catalytique à l'étape i.
2) soit (étape j) couplée au bromure obtenu à l'étape d, pour obtenir la sulfone à longue chaîne. Le groupe phényl sulfone est éliminé à l'étape k.

L'éther de tétrahydropyrannyle du composé A₁ obtenu par les 2 voies de synthèse, étapes i ou j, est déprotégé à l'étape l.

Une hydrogénation catalytique permet de passer du composé A au composé B à l'étape m.

Les composés A₂, A₃, B₂ et B₃ sont synthétisés de manière analogue aux composés A₁ et B₁, par couplage avec les synthons adéquats correspondants.

On va maintenant, à titre d'exemple, donner les modes opératoires détaillés des étapes a à m du processus de synthèse des composés A₁ et B₁ selon l'invention.

Etape a: préparation du 1,10-décanediol monoprotégé: (C₁₅H₃₀0₃=258). On solubilise le 1,10-décanediol (5 g; 28,7 mmoles) dans 20 ml de dioxanne anhydre, et on ajoute 1 éq de dihydropyranne (DHP) et 0,1 éq d'acide *p*-toluènesulfonique (pTsOH). Après 2 heures de réaction, la phase organique est lavée avec une solution saturée de bicarbonate de sodium, de chlorure de sodium, puis séchée avec du sulfate de magnésium et évaporée. Le produit est purifié par chromatographie sur silice (hexane/éther: 65/35). Le rendement est de 27% (2 g; 7,74 mmoles). TLC (hexane/éther: 5/5): Rf=0,34.

Etape b: préparation du 10-(tétrahydropyran-2-oxy)-1-décanal: (C₁₅H₂₈0₃=256). On solubilise l'alcool obtenu à l'étape a (2 g; 7,74 mmoles) dans du dichlorométhane anhydre et on l'ajoute à une solution de chlorochromate de pyridinium (3,3 g; 15,5 mmoles; PCC) dans 100 ml de dichlorométhane à température ambiante pendant 3 heures. Le mélange est filtré ensuite sur colonne de Florisil (éluant: hexane/éther: 1/1) puis purifié par chromatographie sur colonne de silice (hexane/ éther: 9/1). Le rendement est de 70% (1,39 g; 5,42 mmoles).

Etape c: préparation du 10-bromo-1-décanol: (C₁₀H₂₁OBr=237). On solubilise le 1,10-décanediol (12 g; 69 mmoles) dans du cyclohexane (170 ml) et une solution aqueuse d'acide bromhydrique à 48% (170 ml) et on chauffe à reflux pendant 3 heures. Aprés refroidissement on neutralise la phase organique avec une solution saturée de bicarbonate de sodium et on extrait avec de l'hexane. Le bromoalcool est purifié par chromatographie sur colonne de silice (hexane/ acétate d'éthyle: 9/1). Le rendement est de 56% (9,16 g; 38,64 mmoles).

Etape d: préparation du 10-bromo-1-(tétrahydropyran-2-oxy)décane: (C₁₅H₂₈O₂Br=321): même réaction que l'étape b. Le rendement est de 75%.

Etape e: préparation de la sulfone linéaire: (C₁₆H₂₂O₂S=278). On solubilise du phénylsulfonate de sodium (7,55 g; 46 mmoles) dans 60 ml de méthanol anhydre et, à 0°C et à l'abri de la lumière, on ajoute lentement le bromure de géranyle (10 g; 46 mmoles) solubilisé dans 5 ml de méthanol. Après 1 heure de réaction, le milieu est hydrolysé, extrait avec du dichlorométhane, séché avec du sulfate de magnésium et évaporé. Le produit est purifié par chromatographie sur silice (hexane/éther: 90/10). Le rendement est de 61 % (7,80 g; 28,06 mmoles). TLC (hexane/éther: 8/2): Rf=0,28.

Etape f: préparation de la sulfone cyclisée: (C₁₆H₂₂O₂S=278). On solubilise la sulfone obtenue à l'étape e (6,35 g; 22,8 mmoles) dans 10 ml d'acide acétique puis, à 12°C on ajoute de l'acide sulfurique concentré (32 éq, 19,8 ml) et on laisse réagir pendant 30 minutes à cette température. Le milieu réactionnel est lavé avec une solution saturée de bicarbonate de sodium à 2 reprises, extrait avec du dichlorométhane puis séché avec du sulfate de magnésium et évaporé. Le produit est purifié par recristallisation dans l'hexane. Le rendement est de 81% (5,10 g; 18,31 mmoles). TLC (hexane/éther: 5/5): Rf=0,63.

Etape g: préparation de l'hydroxy-sulfone: (C₃₁H₅₀O₅S=534). On solubilise la sulfone cyclisée (200 mg; 0,70 mmoles; 1,1 éq) dans du THF anhydre et quelques gouttes d'HMPT anhydre. A -78°C, on ajoute 1 éq de Butyllithium (0,63 mmoles) et laisse réagir à -78°C pendant 2 heures. On ajoute l'aldéhyde obtenu à l'étape b (162 mg; 0,63 mmoles; 1 éq) solubilisé dans du THF et on laisse réagir pendant 2 heures à -78°C. Le milieu est hydrolysé, extrait 3 fois à l'éther, séché avec du sulfate de magnésium et évaporé. On purifie par chromatographie sur silice (hexane/ éther: 70/30 puis 65/35). Le rendement est de 54% (184 mg; 0,34 mmoles). TLC (hexane/éther: 5/5): Rf=0,32.

Etape h: préparation du diène: (C₂₅H₄₄O₂=376). On solubilise l'hydroxy-sulfone préparée à l'étape g (198 mg; 0,37 mmole) dans 40 ml de méthanol anhydre. A 0°C, on ajoute du Na₂HPO₄ (236 mg; 1,66 mmoles) et de l'amalgame de sodium (6% Hg; 2,37 g). On laisse réagir à 4°C pendant 7 heures avant d'hydrolyser le milieu réactionnel. On extrait 3 fois avec du dichlorométhane et on purifie le produit par chromatographie sur silice (hexane/éther: 9/1). Le rendement est de 70% (98 mg 0,26 mmole). TLC (hexane/éther: 8/2): Rf=0,69.

Etape l: préparation de l'oléfine: (C₂₅H₄₆O₂=378). On solubilise le diène (98 mg; 0,26 mmole) dans 20 ml de méthanol et on ajoute 100 mg de Pd sur charbon sous atmosphère d'hydrogène. Après 8 heures de réaction on filtre sur célite et on évapore le solvant. Le rendement est de 70%. TLC (hexane/éther: 8/2): Rf=0,29.

Etape j: préparation de la sulfone à chaîne longue par couplage avec le bromure préparé à l'étape d: (C₃₁H₅₀O₄S=518). On solubilise la sulfone cyclisée (1,7 g; 6 mmoles) dans du THF anhydre et 1 éq de tBuOK à température ambiante. On ajoute ensuite le bromure (1 mmole) à -30°C. On laisse revenir le milieu réactionnel à température ambiante et on laisse réagir pendant 5 heures. On hydrolyse le milieu et on extrait 3 fois avec l'éther et évapore le solvant. On purifie par chromatographie sur silice (hexane/éther: 9/1). Le rendement est de 60% (1,86 g; 3,60 mmoles). TLC (hexane/éther: 8/2): Rf=0,28.

Etape k: préparation de l'oléfine par désulfonation: (C₂₅H₄₆O₂=378). On solubilise la sulfone préparée à l'étape j (518 mg; 1 mmole) dans 40 ml de méthanol anhydre. A 0°C, on ajoute du Na₂HPO₄ (142 mg; 4,3 mmoles) et de l'amalgame de sodium (6% Hg; 1,8 g). On laisse réagir à température ambiante pendant une nuit avant d'hydrolyser le milieu réactionnel. On extrait 3 fois avec de l'éther et on purifie le produit par chromatographie sur silice (hexane/éther: 9/1). Le rendement est de 99% (374 mg; 0,99 mmole). TLC (hexane/éther: 7/3): Rf=0,72.

Etape l: obtention du composé A₁: (C₂₀H₃₈O=294). On solubilise l'oléfine dans du méthanol à température ambiante pendant 30 minutes avec de l'acide *p*-toluènesulfonique. On évapore le méthanol et on reprend le résidu avec de l'éther. On lave la phase organique avec une solution de NaHCO₃, on séche avec du MgSO₄ et on évapore. On purifie par chromatographie sur silice (hexane/éther: 9/1). Le rendement est de 95%. TLC (hexane/éther: 9/1): Rf=0,15.

Etape m: obtention du composé B₁: (C₂₀H₄₀O=296). On soumet à une hydrogénation catalytique poussée (H₂/Pd/C) dans du méthanol (voir préparation de l'éther de tétrapyrannyle). TLC (hexane/éther: 9/1): Rf=0,15.

### II) Essais biologiques In vitro: différenciation et survie de neurones cérébraux en présence des dérivés de l'invention.

Les dérivés de l'invention ont été testés sur des neurones du système nerveux central en culture primaire. Les cultures sont préparées à partir de cerveaux d'embryons de rats. Les neurones proviennent de l'une des trois zones cérébrales suivantes: le cortex à partir d'embryons de 14 jours, l'hippocampe et le septum à partir d'embryons de 18 jours. Les cellules sont ensemencées à faible densité (10⁵ cellules/lamelle) sur des lamelles prétraitées avec de la poly-L-lysine. Les cellules sont maintenues dans un milieu de culture chimiquement défini. Les dérivés testés sont ajoutés le premier jour de la mise en culture des cellules à des doses variant de 10⁻⁹ M à 10⁻⁴ M. Le changement de milieu se fait tous les quatre jours avec addition des composés à tester.

On suit la survie des neurones en culture à l'aide d'un microscope en contraste de phase. De manière générale, les cellules témoins dégénèrent après 11 jours de culture alors que le nombre de cellules survivantes est significativement élevé dans les cultures traitées avec les dérivés de l'invention.

Après 48 heures de culture, les neurones traités émettent des prolongements plus longs et plus ramifiés, ceci a été démontré par immunocytochimie contre les neurofilaments (marqueur spécifique des neurones). Il a également été montré, par immunocytochimie, l'effet de certains composés de l'invention sur l'expression de la choline acétyltransférase (CAT), marqueur spécifique des neurones cholinergiques. Ces résultats indiquent un effet des composés de l'invention testés sur la différenciation neuronale.

On observe également que les neurones traités émettent des prolongements plus longs et plus ramifiés et forment un réseau intriqué de neurites. Ces résultats montrent une plus grande plasticité du tissu nerveux laquelle *in vivo* correspond à une capacité accrue de régénération et à la mise en place de nouveaux circuits neuronaux.

Les composés testés favorisent la différenciation des trois types de neurones et favorisent la survie des neurones de cortex et de l'hippocampe. L'utilisation de ces modèles de culture permet de mettre en évidence la spécificité d'action des composés sur chaque type de cellule.

Il ressort des résultats obtenus qu'*in vitro* les dérivés de l'invention ont un effet comparable à celui de certains facteurs neurotrophiques protéiques tel que le facteur de croissance des nerfs (NGF).

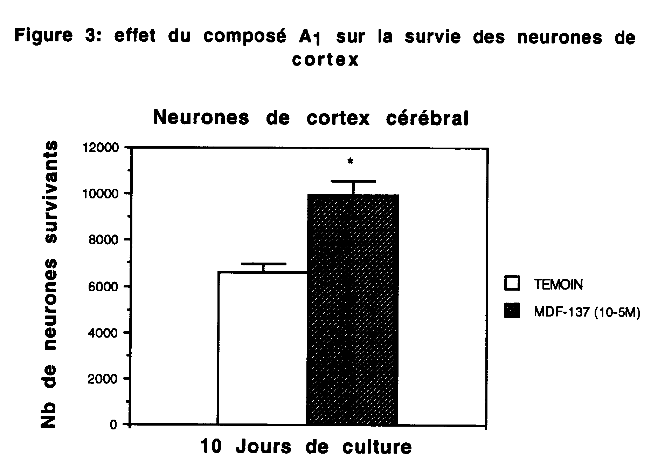

A titre d'exemple, l'effet du composé A₁, sur la survie des neurones de cortex est représenté sur la **figure 3**. Ce graphique représente le nombre de neurones survivants après 10 jours de culture. Le nombre de neurones survivants dans les cultures traitées par le composé A₁ (10⁻⁵M) est significativement augmenté par rapport aux cellules témoins (t(2) = 6,75; * p < 0,02, t de Student).

Par ailleurs les propriétés décrites ci-dessus ne sont pas limitées aux neurones d'origine cérébrale, mais s'appliquent également au système nerveux périphérique. Ainsi une lésion des nerfs périphériques entraîne une dégénérescence axonale, appelée dégénérescence wallérienne. Il peut y avoir perte de la continuité du nerf et donc de la fonction de l'organe normalement innervé. Les composés de l'invention favorisent la régénérescence de la partie proximale du nerf lésé, ce qui a pour effet de permettre la réinnervation de l'organe terminal. Cet action permet de rétablir la fonction de l'organe réinnervé grâce au traitement à l'aide de ce composé.

### III) Essais biologiques in vivo: activités neurotrophique et promnésiante des composés de l'invention

### 1) Activité neurotrophique in vivo: Effet de restauration fonctionnelle

Les molécules entrant dans le cadre de l'invention ont une activité neurotrophique *in vivo.* D'un point de vue morphologique, ces molécules ont un effet de différenciation et un effet de survie neuronale dans divers modèles de lésion. D'un point de vue fonctionnel, les composés de l'invention ont un effet de restauration comportementale et biochimique, notamment sur le modèle de la lésion du noyau basal magnocellulaire (NBM) chez le rat (Dubois *et al*., 1985). L'activité des composés de l'invention peut être démontrée à l'aide du modèle expérimental décrit ci-dessous.

On observe que les rats porteurs de la lésion du NBM et ayant reçu un traitement avec un composé de l'invention ne développent pas d'hyperactivité locomotrice nocturne, par comparaison au groupe NBM/Témoin. Pendant 13 jours, des rats non lésés (NL) ou porteurs de la lésion du NBM (NBM) sont traités par voie intrapéritonéale ou orale soit avec une solution témoin, constituée par le solvant seul, soit avec un_composé de l'invention. Deux jours après la fin du traitement (soit 12 jours après la lésion), l'activité locomotrice nocturne est enregistrée dans des actimètres automatisés pendant 9 heures.

Les rats porteurs de la lésion du NBM ayant reçu un traitement avec un composé de l'invention ne présentent pas de déficit d'apprentissage d'une réponse d'évitement passif. Pendant 13 jours, des rats non lésés (NL) ou porteurs de la lésion du NBM (NBM) sont traités par voie intrapéritonéale ou orale soit avec une solution témoin, constituée par le solvant seul, soit avec un composé de l'invention. Neuf jours après la fin du traitement (soit 21 jours après la lésion), les rats sont soumis à l'apprentissage d'une réponse d'évitement passif. Au cours d'un essai, le rat est placé au bout d'une allée étroite et fortement illuminée conduisant à un large compartiment obscur. Il doit apprendre à rester passivement sur l'allée pendant un délai minimum de 300 secondes (critère d'apprentissage); chaque entrée dans le compartiment, dont la latence est mesurée en secondes, est punie par la délivrance d'un choc électrique (0,5 mA pendant 5 s). La séance d'apprentissage comporte autant d'essais nécessaires à l'obtention du critère. On note un déficit d'apprentissage dans le groupe NBM/Témoin et une courbe d'apprentissage du groupe NBM/composé similaire à celle des groupes NL.

### 2) Activité promnésiante

L'activité promnésiante des molécules entrant dans le cadre de l'invention est mise en évidence à l'aide de plusieurs épreuves comportementales sollicitant la mémoire spatiale de travail, de référence, à court terme (comportement d'alternances spontanées ou renforcées, discrimination spatiale dans un labyrinthe en T), la mémoire spatiale à long terme (reconnaissance d'un environnement initialement nouveau; rétention d'une réponse d'évitement passif), la mémoire olfactive (mémoire sociale d'un jeune congénère), visuelle (comparaison non différée d'échantillons). Dans tous les cas, une facilitation de la mise en mémoire est observée chez les animaux ayant reçu par voie orale ou intra-péritonéale une solution contenant ces molécules.

Si l'on mesure le comportement d'alternances spontanées dans un labyrinthe en T, les rats ayant reçu cet alcool gras à chaîne longue ont un pourcentage d'alternances spontanées plus élevé que ceux traités avec la solution témoin; ce qui indique une facilitation de la mémoire de travail à court terme.

Des rats reçoivent pendant 10 jours des injections intrapéritonéales quotidiennes d'une solution témoin ou d' un composé de l'invention. Le jour de la dernière injection, chaque animal est soumis à une séance d'habituation de 10 minutes pendant lesquelles il peut librement explorer le labyrinthe en T. Cinq jours après, le comportement d'alternances spontanées est mesuré au cours de 11 essais successifs.

On note une facilitation du comportement d'alternances spontanées cinq jours après la fin d'un traitement avec un composé de l'invention .

En outre, l'effet promnésiant des composés de l'invention est généralisable à plusieurs formes de mémoire (mémoire à court/long terme; mémoire de travail/référence; mémoire spatiale, olfactive, visuelle). Cet effet apparaît après un traitement répété ou unique administré par voie orale ou intra-péritonéale.

### 3) Greffes nerveuses

Un traitement pharmacologique n'est pas le seul moyen qui permette de favoriser une récupération fonctionnelle à la suite d'une lésion du système nerveux central. En effet il est possible de remplacer certains neurones ou de compenser un déficit en neurotransmetteur à l'aide d'une greffe nerveuse. Le tissu greffé consiste le plus souvent en tissu nerveux foetal. La greffe peut agir à plusieurs niveaux pour stimuler la récupération fonctionnelle. Elle peut reconstituer un circuit qui a été interrompu par une lésion ou peut fournir des neurotransmetteurs nécessaires au fonctionnement de certains circuits neuronaux.

Les composés de l'invention sont capables de favoriser la prise d'une greffe nerveuse grâce à leur capacité à stimuler la survie et la différenciation neuronale. En particulier l'extension de neurites à partir du tissu nerveux greffé favorise sa mise en contact avec les tissus environnants et donc le fonctionnement du circuit neuronal. Par ailleurs une bonne survie neuronale au sein de la greffe est nécessaire afin d'assurer son fonctionnement à long terme.

De ces essais *in vivo,* il ressort clairement que les composés de l'invention peuvent être utilisés comme neurotrophiques dans les maladies neurodégénératives grâce à leur effet de récupération fonctionnelle à la suite de lésions du tissu nerveux mais aussi comme nootropes, grâce à leur effet facilitateur des capacités mnésiques .

*In vitro,* les dérivés de l'invention favorisent la différenciation et la survie neuronale. *In vivo,* ils sont capables de compenser le déficit de mémoire après lésion cérébrale.

Les dérivés de l'invention peuvent donc être avantagement utilisés dans certaines maladies neurodégénératives. Leur bonne biodisponibilité et l'obtention de produits chimiquement et optiquement purs en feront des outils thérapeutiques particulièrement utiles.

### Référence bibliographique

Dubois B., Mayo W., Agid Y., Le Moal M. and Simon H. (1985) - Profound disturbances of spontaneous and learned behaviors following lesions of the nucleus basalis magnocellularis in the rat. Brain Research, 338, 249-258.

## Revendications

1. Nouveaux dérivés d'alcools gras à longue chaîne, caractérisés par les formules générales suivantes: 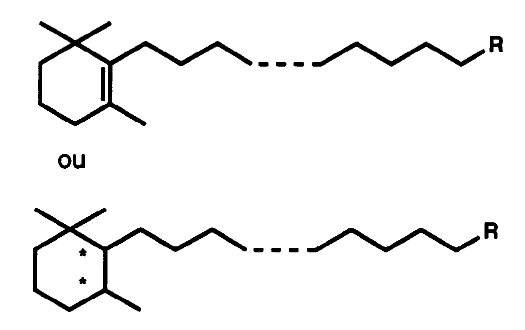 avec
**R**= -CH₂CH₂CH₂OH
ou
**R**= -CH=CHCH₂OH, *cis* ou *trans*
comportant une chaîne hydrocarbonée linéaire de 7 à 25 atomes de carbone saturée ou comportant une double liaison de configuration *cis* ou *trans* en β de la fonction alcool, mais sans aucune ramification.

2. Dérivés selon la revendication 1, caractérisés en ce que la chaîne linéaire compte 11 atomes de carbone sans ou avec une double liaison de configuration *cis ou trans* en β de la fonction alcool.

3. Compositions pharmaceutiques caractérisées en ce qu'elles comprennent au moins un dérivé selon l'une des revendications 1 et 2, à une dose efficace de l'ordre de 0,1-100 mg/kg (administration unique ou répétée), de préférence 5-50 mg/kg, ladite composition étant administralle par voie topique, orale, intra-cérébrale, intra-musculaire, intra-veineuse ou parentérale, la composition comprenant en outre une quantité adéquate d'un excipient physiologiquement acceptable.

4. Compositions selon la revendication 3, pour utilisation dans le traitement des maladies neurodégénératives telles que la maladie d'Alzheimer.

5. Compositions selon la revendication 3, pour utilisation dans le traitement de toute situation entraînant une diminution des capacités d'apprentissage et de mémoire et en particulier au cours du vieillissement cérébral.

6. Compositions selon la revendication 3, pour utilisation pour favoriser la récupération fonctionnelle à la suite de lésions du tissu nerveux.

7. Utilisation des compositions selon la revendication 3, pour améliorer les fonctions cognitives d'un sujet sain.

8. Compositions selon la revendication 3,pour utilisation pour favoriser la prise de greffes de tissu nerveux d'origine foetale ou adulte.

9. Compositions selon la revendication 3, pour utilisation pour favoriser la régénération de nerfs périphériques, en particulier à la suite de lésions traumatiques ou neuropathiques.

## Claims

1. New long chain fatty alcohols derivatives characterized by the general formula: 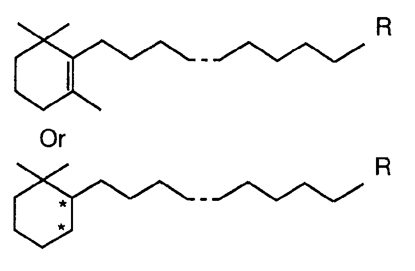 with
**R**=-CH₂CH₂CH₂OH
or
**R**=-CH=CHCH₂OH, *cis* or *trans*
in which the side chain is a linear hydrocarbon chain with 7 to 25 carbon atoms, saturated or with a double bond with *cis* or *trans* configuration in the β position from the primary alcohol function, and without ramifications.

2. Derivatives as in claim 1, characterized in that said linear side chain has 11 carbon atoms, with or without a *cis* or *trans* double bond in β position from the primary alcohol function.

3. Pharmaceutical compositions characterized in that they comprise at least one of the derivatives as in claims 1 or 2, at an efficient dose of **((0.1-100** **mg/kg))** (single or multiple administrations), especially **((5-50 mg/kg))**, said compositions being administrated topically, orally, intra-cerebrally, intra-musculary, intraveneously or intra peritoneally, said compositions comprising also a proper amount of a physiological acceptable excipient.

4. Compositions according to claim 3, for the treatment of neurodegenerative diseases like Alzheimer's disease.

5. Compositions according to claim 3, for the treatment of any affection which causes a decrease of learning capacities and memory, in particular during cerebral aging.

6. Compositions according to claim 3, to favour functional recovery following lesions of the nervous tissue.

7. Compositions according to claim 3, to improve cognitive functions of normal individuals.

8. Compositions according to claim 3, to favour nervous tissue grafts from foetal or adult origin.

9. Compositions according to claim 3, to favour the regeneration of peripheral nerves, in particular following traumatic lesions or neuropathies.

## Patentansprüche

1. Neue Derivate langkettiger Fettalkohole, gekennzeichnet durch die folgenden allgemeinen Formeln: 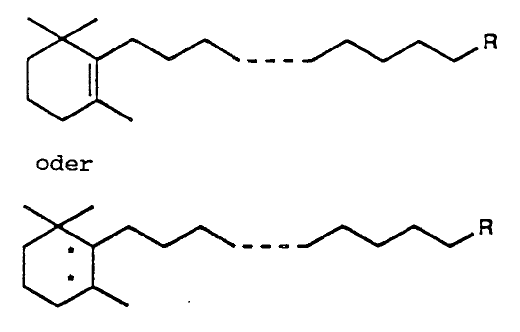 mit
**R**= -CH₂CH₂CH₂OH
oder
**R**= -CH=CHCH₂OH, cis oder trans
die eine geradkettige Kohlenwasserstoffkette mit 7 bis 25 Kohlenstoffatomen besitzen, die gesättigt ist oder eine Doppelbindung in cis- oder trans-Konfiguration an der β-Stellung der Alkoholfunktion besitzen, aber nicht verzweigt sind.

2. Derivate nach Anspruch 1, dadurch gekennzeichnet, daß die lineare Kette 11 Kohlenstoffatome enthält und keine oder eine Doppelbindung in cis- oder trans-Konfiguration an der β-Stellung zur Alkoholfunktion.

3. Pharmazeutische Zusammensetzungen, dadurch gekennzeichnet, daß sie mindestens ein Derivat nach einem der Ansprüche 1 und 2 umfassen, in einer wirksamen Dosis in der Größenordnung von 0.1 bis 100 mg/kg (bei einmaliger oder wiederholter Verabreichung), und vorzugsweise von 5-50 mg/kg, und die Zusammensetzung auf topischem, oralem, intracerebralem, intramuskulärem, intravenösem oder parenteralem Weg verabreicht wird, und die Zusammensetzung außerdem eine entsprechende Menge eines physiologisch annehmbaren Trägers umfaßt.

4. Zusammensetzungen nach Anspruch 3 zur Verwendung zur Behandlung neurodegenerativer Erkrankungen, wie z.B. der Alzheimer Krankheit.

5. Zusammensetzungen nach Anspruch 3 zur Verwendung zur Behandlung von Zuständen, die eine Verringerung der Lernfähigkeit und des Gedächtnisses mit sich bringen und insbesondere während des Abbaus des Gehirns.

6. Zusammensetzungen nach Anspruch 3 zur Verwendung zur Förderung der funktionellen Wiederherstellung nach Läsionen des Nervengewebes.

7. Verwendung von Zusammensetzungen nach Anspruch 3 zur Verbesserung der cognitiven Funktionen eines gesunden Lebewesens.

8. Zusammensetzungen nach Anspruch 3 zur Verwendung zur Förderung der Aufnahme von transplantiertem Nervengewebe foetalen Ursprungs oder von Erwachsenen.

9. Zusammensetzungen nach Anspruch 3 zur Verwendung zur Förderung der Regenerierung peripherer Nerven, und insbesondere nach traumatischen oder neurpathischen Läsionen.
